# FASCICULE DE BREVET EUROPEEN

(11) **EP 3 184 610 B1**
(45) Date de publication et mention de la délivrance du brevet: **20.02.2019**
(21) Numéro de dépôt: 16306612.9
(22) Date de dépôt: 02.12.2016
(51) Int. Cl.: C10G 65/02, C10G 45/32, B01J 8/04

(54) **PROCÉDÉ D'HYDROGENATION SELECTIVE DE CHARGES OLEFINIQUES AVEC UN SEUL REACTEUR PRINCIPAL ET UN REACTEUR DE GARDE DE TAILLE REDUITE**
SELEKTIVES HYDRIERVERFAHREN VON OLEFINISCHEN LADUNGEN MIT EINEM EINZIGEN HAUPTREAKTOR UND EINEM SCHUTZREAKTOR GERINGERER GRÖSSE
METHOD FOR SELECTIVE HYDROGENATION OF OLEFIN FEEDSTOCKS WITH A SINGLE MAIN REACTOR AND A COMPACT GUARD REACTOR

(30) Priorité: 22.12.2015 FR 1563022
(43) Date de publication de la demande: 28.06.2017
(73) Titulaire: IFP Energies nouvelles, 92500 Rueil-Malmaison (FR)
(72) Inventeur: CHARRA, Cyprien, 69003 LYON (FR); MEKKI-BERRADA, Adrien, 42000 ST ETIENNE (FR); BAZER-BACHI, Frederic, 30340 SAINT PRIVAT DES VIEUX (FR); FISCHER, Beatrice, 69005 LYON (FR); HILLY, Florian, 92200 NEUILLY SUR SEINE (FR)
(74) Mandataire: IFP Energies nouvelles

(56) Documents cités:
- FR-A1- 2 784 687
- FR-A1- 2 810 991
- FR-A1- 2 970 260
- FR-A1- 2 984 915
- US-A- 4 118 310

## Description

La présente invention a pour objet un procédé d'hydrogénation sélective d'une charge d'hydrocarbures contenant des molécules polyinsaturées comprenant au moins 3 atomes de carbone et sélectionnée dans le groupe constitué par une coupe C3 de vapocraquage, une coupe C4 de vapocraquage, une coupe C5 de vapocraquage et une essence de pyrolyse et de leur mélange, utilisant un seul réacteur d'hydrogénation principal à lit fixe contenant au moins deux lits catalytiques et un réacteur d'hydrogénation de garde à lit fixe de taille réduite, lesdits réacteurs d'hydrogénation étant disposés en série pour être utilisés de façon cyclique selon une séquence d'étapes permettant de court-circuiter le ou les lits catalytiques au moins partiellement désactivés du réacteur principal à l'aide du réacteur de garde tout en assurant le fonctionnement du procédé en continu.

Les composés organiques mono-insaturés, tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent une grande variété de molécules monoinsaturées recherchées telles que l'éthylène, le propylène, les butènes linéaires, l'isobutène, les pentènes ainsi que des molécules insaturées contenant jusqu'à environ 15 atomes de carbone.

En parallèle, des composés polyinsaturés tels que l'acétylène, le propadiène et le méthylacétylène (ou propyne), le 1-2 butadiène et le 1-3 butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés styréniques ou indéniques, sont également formés. Ces composés polyinsaturés sont très réactifs et conduisent à des réactions parasites dans les unités de polymérisation. Il est donc nécessaire de les éliminer avant de valoriser ces coupes. Dans le cas d'une charge d'essence de pyrolyse, ces composés doivent être éliminés avant le traitement d'hydrodésulfuration classiquement effectué par la suite.

L'hydrogénation sélective est le principal traitement développé pour éliminer spécifiquement les composés polyinsaturés indésirables de ces charges d'hydrocarbures.

L'hydrogénation sélective des hydrocarbures insaturés permet d'hydrogéner sélectivement les composés polyinsaturés présents dans la charge à traiter de manière à ce que les composés dioléfiniques ou acétyléniques soient partiellement hydrogénés en mono-oléfines et que les composés styréniques et indéniques présents dans les coupes essences, soient partiellement hydrogénés en composés aromatiques correspondants en évitant leur saturation totale de manière à éviter la formation des alcanes ou naphtènes correspondants.

Les unités conventionnelles d'hydrogénation sélective d'hydrocarbures insaturés comprennent généralement une section principale d'hydrogénation comprenant un réacteur catalytique à lit fixe dans lequel les charges hydrocarbures liquides sont mises en contact avec de l'hydrogène gazeux (réacteur bi-phasique, ou monophasique lorsque tout l'hydrogène peut être dissous dans la charge).

En parallèle de ces réactions d'hydrogénations sélectives, des réactions secondaires peuvent mener à la formation d'oligomères pouvant conduire au dépôt de gommes à la surface du catalyseur, entrainant sa désactivation progressive. De plus, les impuretés contenues dans la charge peuvent entraîner une désactivation du catalyseur. Il en résulte que le catalyseur doit être régénéré pour recouvrer son efficacité. C'est pourquoi les unités conventionnelles d'hydrogénation sélective comprennent généralement un second réacteur à lit fixe vers lequel la réaction d'hydrogénation est redirigée lorsque le premier réacteur doit être réactivé ou régénéré.

Le document FR 2 984 915 décrit un procédé d'hydrogénation sélective d'une charge oléfinique insaturée comprenant 3 ou 4 atomes de carbone, utilisant au moins deux réacteurs principaux permutables en lit fixe contenant chacun au moins un lit catalytique et dans lequel ladite charge oléfinique traverse successivement tous les réacteurs, et dans lequel, chaque fois que l'un des réacteurs est désactivé, le point d'introduction de la charge est déplacé vers l'aval. Ce document propose donc d'optimiser les installations d'hydrogénation sélective d'hydrocarbures insaturés par l'utilisation des réacteurs disponibles en série dans la section principale d'hydrogénation, sans pour autant devoir interrompre le procédé pour la régénération des catalyseurs.

Cependant, le procédé décrit dans FR 2 984 915 nécessite au moins deux réacteurs principaux de taille identique dans lequel un seul des réacteurs procède à l'hydrogénation pendant que le second (aussi appelé « spare » en terminologie anglo-saxonne) est régénéré. Ce second réacteur, bien que présent dans l'installation, n'est donc pas utilisé pour l'hydrogénation de la charge tant que le catalyseur du premier réacteur n'est pas désactivé.

La présente invention propose d'optimiser les procédés d'hydrogénation sélective d'hydrocarbures insaturés en proposant d'utiliser qu'un seul réacteur principal et un réacteur de garde de taille plus petite, lesdits réacteurs étant disposés en série pour être utilisés de façon cyclique selon une séquence d'étapes permettant de court-circuiter le ou les lit catalytiques au moins partiellement désactivés du réacteur principal à l'aide du réacteur de garde tout en assurant le fonctionnement du procédé en continu.

Ainsi, la présente demande a pour objet un procédé d'hydrogénation sélective selon la revendication 1.

Le procédé d'hydrogénation sélective selon l'invention ne nécessite ainsi pas de deuxième réacteur principal ce qui représente une économie de coût d'investissement et de coût de fonctionnement. En effet, bien que le procédé de l'invention nécessite un réacteur de garde, celui-ci est de taille réduite par rapport à un deuxième réacteur principal.

Par rapport à un procédé d'hydrogénation sélective fonctionnant à un seul réacteur principal (sans réacteur de garde de taille réduite), le procédé selon l'invention permet d'augmenter la capacité de traitement de la charge ainsi qu'une augmentation du cycle de temps, pour un même volume catalytique. De plus, aucun arrêt lors de la régénération du catalyseur d'un des réacteurs n'est nécessaire. Ce procédé est donc particulièrement utile lorsque l'on souhaite augmenter la capacité d'unités existantes (« revamping » en terminologie anglo-saxonne) de manière économique. Le procédé selon l'invention permet d'accroître la durée des temps de cycle en retardant la formation d'oligomères (ou gommes), reportant d'autant la désactivation du catalyseur.

De plus, le procédé d'hydrogénation sélective selon l'invention permet d'amener de la flexibilité lors de l'opération, tant en terme de coupes plus difficiles à traiter que de changements de capacité.

Selon une variante, le volume total du catalyseur contenu dans le ou les lits catalytiques du réacteur de garde est au maximum 60 % du volume total du catalyseur contenu dans les lits catalytiques du réacteur principal.

La charge d'hydrocarbures est sélectionnée dans le groupe constitué par une coupe C3 de vapocraquage, une coupe C4 de vapocraquage, une coupe C5 de vapocraquage et une essence de pyrolyse et de leur mélange.

La durée de fonctionnement pour chacune des étapes a), b), c) et d), défini par rapport à une durée maximale de fonctionnement tₘₐₓ pour chaque étape qui est le temps pour atteindre une valeur maximalement tolérable d'une température ou d'une sélectivité pour une charge donnée, est respectivement :
- pour l'étape a) : entre 10 et 70 % de t_{Amax}
- pour l'étape b) : entre 40 et 100 % de t_{Bmax}
- pour l'étape c) : entre 90 et 100 % de t_{Cmax}
- pour l'étape d) : entre 70 et 100 % de t_{Dmax}.

La valeur maximalement tolérable d'une charge C3 est la sélectivité correspondant à l'atteinte d'une égalité de composition en propylène entre l'entrée et la sortie du réacteur en fin de cycle, la valeur maximalement tolérable d'une charge C4 est une température maximale de 160°C, la valeur maximalement tolérable d'une charge C5 est une température maximale de 160 °C, la valeur maximalement tolérable d'une charge d'essence de pyrolyse est une température maximale de 200°C.

Selon une variante, chaque réacteur est mis en oeuvre à une température allant de 0°C à 200°C, à une pression comprise entre 1 et 6,5 MPa et à une vitesse volumique horaire globale, définie comme le rapport du débit volumique de la charge fraîche à 15°C sur le volume total de catalyseur présent dans l'ensemble des réacteurs mis en oeuvre, comprise entre 1 h⁻¹ et 100 h⁻¹.

Selon une variante, une partie de l'effluent issue du réacteur principal ou du réacteur de garde est recyclée en mélange avec la charge à hydrogéner.

Selon une variante, les conditions opératoires d'hydrogénation durant les étapes a) b) c) et d) sont identiques.

Selon une autre variante, durant l'étape d) la température du réacteur de garde est augmentée et/ou le flux de la phase comprenant de l'hydrogène est augmenté, et/ou le débit de recycle dans le réacteur de garde est augmenté et/ou le débit de charge introduit dans le réacteur de garde est diminué par rapport à respectivement la température, le flux ou le(s) débit(s) utilisé(s) en début de l'étape a).

Selon une variante, l'augmentation de la température du réacteur de garde en tête de réacteur au début de l'étape d) par rapport au début de l'étape a) est comprise entre 0,5 et 40°C.

Selon une variante, dans lequel l'augmentation du débit de recycle au réacteur de garde au début de l'étape d) par rapport au débit de recycle en début de l'étape a) est comprise entre 0,5 et 100%.

Selon une variante, une trempe est introduite entre deux lits catalytiques dans le réacteur principal ou le réacteur de garde, ladite trempe pouvant être une trempe liquide et/ou une trempe gazeuse.

Selon une variante, on met en oeuvre un ou plusieurs échangeur(s) de chaleur entre le réacteur de garde et le réacteur principal.

Selon une variante, les catalyseurs d'hydrogénation utilisés sont identiques ou différents dans le ou les lits catalytiques du réacteur principal et/ou du réacteur de garde.

### Description détaillée

La présente invention a pour objet un procédé d'hydrogénation sélective d'une charge d'hydrocarbures selon la revendication 1.

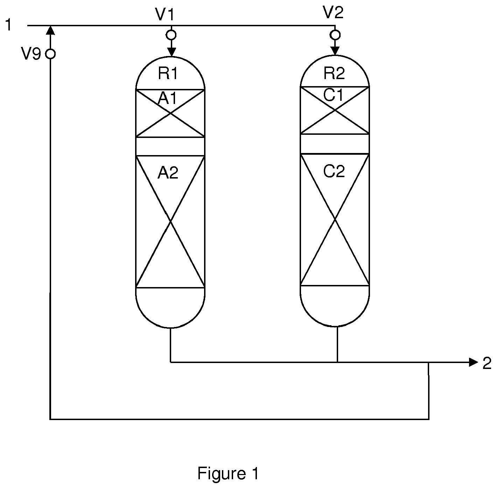

Les procédés classiques d'hydrogénation sélective comprennent, comme le montre la figure 1, deux réacteurs d'hydrogénation : un premier réacteur principal R1 contenant les lits catalytiques A1 et A2, et en parallèle un deuxième réacteur principal de taille identique R2 contenant les lits catalytiques C1 et C2.

Dans ce procédé, une charge d'hydrocarbures est mélangée à une phase gazeuse comprenant de l'hydrogène et ce mélange 1 est introduit en tête du réacteur d'hydrogénation R1 opéré dans les conditions d'hydrogénation. La vanne V1 est ainsi ouverte, et la vanne V2 est, quant à elle, fermée. La charge traverse successivement les lits catalytiques A1 et A2 et on récupère en fond du réacteur R1 l'effluent 2 sélectivement hydrogéné.

Une partie de la phase liquide contenant la charge oléfinique hydrogénée peut être recyclée, après avoir été éventuellement refroidie, via la vanne V9 à l'entrée du réacteur R1, pour être mélangée, avec la charge à hydrogéner, à la phase gazeuse comprenant l'hydrogène.

Lorsque le réacteur R1 commence à se désactiver, la charge est orientée vers le deuxième réacteur R2. Pour cela, la vanne V2 est ouverte, et la vanne V1 est fermée. La charge est ainsi traitée dans le réacteur R2 et traverse successivement les lits catalytiques C1 et C2 pour récupérer en fond du réacteur R2 l'effluent 2 sélectivement hydrogéné pendant que R1 est nettoyé, et son catalyseur régénéré. De la même manière, une partie de la phase liquide contenant la charge oléfinique hydrogénée peut être recyclée, après avoir été éventuellement refroidie, via la vanne V9 à l'entrée du réacteur R2 pour être mélangée, avec la charge à hydrogéner et la phase gazeuse comprenant l'hydrogène.

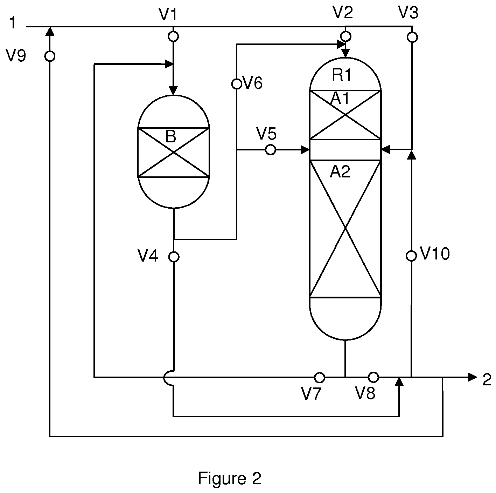

Dans le cadre du procédé d'hydrogénation sélective selon l'invention, tel qu'illustré à la figure 2, on fait passer la charge et une phase gazeuse comprenant de l'hydrogène, dans des conditions d'hydrogénation, sur un catalyseur d'hydrogénation, dans un seul réacteur principal à lit fixe contenant au moins deux lits catalytiques et un réacteur de garde à lit fixe de taille plus petite contenant au moins un lit catalytique, lesdits réacteurs étant disposés en série pour être utilisés de façon cyclique en répétant successivement des étapes a), b), c), c'), d) et d') définies ci-après :
- une étape a) durant laquelle la charge traverse successivement tous les lits catalytiques du réacteur principal,
- lorsque le premier lit catalytique du réacteur principal commence à se désactiver, une étape b) durant laquelle la charge est introduite dans le réacteur de garde puis, en court-circuitant le premier lit catalytique partiellement désactivé du réacteur principal, dans le prochain lit catalytique non désactivé dudit réacteur principal situé immédiatement en aval par rapport au sens de circulation de la charge,
- une étape c) durant laquelle la charge traverse uniquement et successivement tous les lits catalytiques du réacteur principal,
- une étape c'), simultanée à l'étape c), durant laquelle le catalyseur désactivé du ou des lits catalytiques du réacteur de garde est régénéré et/ou remplacé par du catalyseur frais,
- une étape d) durant laquelle la charge ne traverse que le réacteur de garde,
- une étape d'), simultanée à l'étape d), durant laquelle le catalyseur désactivé des au moins deux lits catalytiques du réacteur principal est régénéré et/ou remplacé par du catalyseur frais.

Au cours de l'étape a) du procédé, la charge, préalablement mélangée à la phase gazeuse comprenant de l'hydrogène, est introduite par la ligne comportant une vanne V2 ouverte vers le réacteur R1. Durant cette période les vannes V1, V3, V4, V5, V6 et V7 sont fermées. La charge traverse successivement les lits catalytiques A1 et A2 et on récupère en fond du réacteur R1 l'effluent 2 sélectivement hydrogéné qui est évacué par la conduite comportant une vanne V8 ouverte.

Une partie de la phase liquide contenant la charge oléfinique hydrogénée peut être recyclée, après avoir été éventuellement refroidie, via la vanne V9 à l'entrée du réacteur R1 pour être mélangée, avec la charge à hydrogéner, à la phase gazeuse comprenant l'hydrogène.

Selon une autre variante, une partie de la phase liquide contenant la charge oléfinique hydrogénée, après avoir été éventuellement refroidie, peut être recyclée via la vanne V10 à l'entrée du lit catalytique A2.

Au fur et à mesure, les lits catalytiques, et notamment le premier lit catalytique A1, vont se désactiver lors de l'étape a). Lorsque le premier lit catalytique A1 commence à se désactiver, on procède au basculement de l'étape a) vers l'étape b). Ce basculement se fait après une durée de fonctionnement de l'étape a) qui se situe entre 10 et 70 %, de préférence entre 20 et 60 % de la durée maximale de fonctionnement t_{Amax} de l'étape a) pour atteindre la valeur maximalement tolérable, telle que définie ci-dessous.

Au cours de l'étape b) du procédé, la charge, préalablement mélangée à la phase gazeuse comprenant de l'hydrogène, est introduite par la ligne comportant une vanne V1 ouverte vers le réacteur de garde B. L'effluent du réacteur de garde, partiellement hydrogéné, est ensuite introduit dans le réacteur principal en amont du lit catalytique A2 par la conduite comportant une vanne V5 ouverte. Durant cette période les vannes V2, V3, V4 et V6 sont fermées. Le lit catalytique A1, partiellement désactivée durant l'étape a), est ainsi court-circuité. La charge traverse ensuite le lit catalytique A2 et on récupère en fond du réacteur principal R1 l'effluent 2 sélectivement hydrogéné qui est évacué par la conduite comportant une vanne V8 ouverte.

Une partie de la phase liquide contenant la charge oléfinique hydrogénée peut être recyclée, après avoir été éventuellement refroidie, via la vanne V7, ou via la vanne V9, à l'entrée du réacteur de garde B, pour être mélangée, avec la charge à hydrogéner, à la phase gazeuse comprenant l'hydrogène.

Selon une autre variante, une partie de la phase liquide contenant la charge oléfinique hydrogénée, après avoir été éventuellement refroidie, peut être recyclée via la vanne V10 à l'entrée du lit catalytique A2.

Au fur et à mesure, le réacteur de garde B étant mis en contact avec la charge va se désactiver lors de l'étape b). Lorsque le catalyseur du ou des lits catalytiques du réacteur de garde est complètement désactivé, on procède au basculement de l'étape b) vers l'étape c). Ce basculement se fait après une durée de fonctionnement de l'étape b) qui se situe entre 40 et 100 %, de préférence entre 70 et 100 % de la durée maximale de fonctionnement t_{Bmax} de l'étape b) pour atteindre la valeur maximalement tolérable, telle que définie ci-dessous.

Au cours de l'étape c) du procédé, la charge, préalablement mélangée à la phase gazeuse comprenant de l'hydrogène, est introduite par la ligne comportant une vanne V2 ouverte vers le réacteur R1. Durant cette période les vannes V1, V3, V4 V5, V6 et V7 sont fermées. La charge traverse successivement les lits catalytiques A1 et A2 et on récupère en fond du réacteur R1 l'effluent 2 sélectivement hydrogéné qui est évacué par la conduite comportant une vanne V8 ouverte. Au fur et à mesure, les lits catalytiques, et notamment le premier lit catalytique A1 étant mis en contact avec la charge continuent à se désactiver.

Selon une variante, une partie de la phase liquide contenant la charge oléfinique hydrogénée peut être recyclée, après avoir été éventuellement refroidie, via la vanne V9 à l'entrée du réacteur R1, pour être mélangée, avec la charge à hydrogéner, à la phase gazeuse comprenant l'hydrogène.

Selon une autre variante, une partie de la phase liquide contenant la charge oléfinique hydrogénée, après avoir été éventuellement refroidie, peut être recyclée via la vanne V10 à l'entrée du lit catalytique A2.

Au fur et à mesure, les lits catalytiques du réacteur principal, et notamment le premier lit catalytique A1, vont se désactiver lors de l'étape c). Lorsque le premier lit catalytique A1 est complètement désactivé, on procède au basculement de l'étape c) vers l'étape d). Ce basculement se fait après une durée de fonctionnement de l'étape c) qui se situe entre 90 et 100 %, de préférence 100 % de la durée maximale de fonctionnement t_{Cmax} de l'étape c) pour atteindre la valeur maximalement tolérable, telle que définie ci-dessous.

Au cours de l'étape c') du procédé, qui est effectuée simultanément à l'étape c), le catalyseur désactivé du ou des lits catalytiques du réacteur de garde B est régénéré et/ou remplacé par du catalyseur frais.

Au cours de l'étape d) du procédé, la charge, préalablement mélangée à la phase gazeuse comprenant de l'hydrogène, est introduite par la ligne comportant une vanne V1 ouverte vers le réacteur de garde B. On récupère en fond du réacteur de garde B l'effluent 2 sélectivement hydrogéné qui est évacué par la conduite comportant une vanne V4 ouverte. La charge ne traverse ainsi que le réacteur de garde. Durant cette période les vannes V2, V3, V5, V6, V7, V8 et V10 sont fermées.

Selon une variante, une partie de la phase liquide contenant la charge oléfinique hydrogénée peut être recyclée, après avoir été éventuellement refroidie, via la vanne V9 à l'entrée du réacteur de garde, pour être mélangée, avec la charge à hydrogéner, à la phase gazeuse comprenant l'hydrogène.

Afin d'assurer une hydrogénation sélective dans le volume de catalyseur réduit dans le ou les lits catalytiques du réacteur B durant cette étape, les conditions d'opérations sont de préférence plus sévères que les conditions d'opérations utilisées en début de l'étape a) (ou les conditions d'opérations utilisées en début des autres étapes b) et c) qui sont généralement identiques à celles de l'étape a). La nécessité de modifier ou non les conditions opératoires dépend notamment de la nature de la charge. Par exemple, plus la charge est lourde ou chargée en impuretés, plus les conditions opératoires doivent être sévérisées.

Selon une variante, et pour sévériser les conditions opératoires et/ou améliorer la maîtrise thermique, on peut :
- augmenter la température du réacteur de garde B durant l'étape d) et/ou
- augmenter le flux de la phase comprenant de l'hydrogène durant l'étape d) et/ou
- diminuer le débit de charge et/ou
- augmenter le débit de recycle.

Selon une variante, on augmente la température du réacteur de garde B. Dans ce cas, l'augmentation de la température du réacteur de garde B en tête de réacteur au début de l'étape d) par rapport au début de l'étape a) est comprise entre 0,5 et 40°C, de préférence entre 3 et 20°C.

Selon une autre variante, il est également possible de diminuer le débit de charge introduit dans le réacteur de garde durant l'étape d).

Selon encore une autre variante, on peut augmenter le débit de recycle pour améliorer la maîtrise thermique. L'augmentation du débit de recycle au début de l'étape d) est compris entre 0,5 et 100% par rapport au débit de recycle au réacteur principal en début de l'étape a), de préférence entre 3 et 50%. L'augmentation du débit de recycle peut être effectuée en plus des autres mesures de sévérisation, telles que l'augmentation de température.

De manière préférée, on augmente la température et/ou on augmente le débit de recycle.

Lorsque le catalyseur du réacteur de garde B est complètement désactivé, on procède au basculement de l'étape d) vers l'étape a). Ce basculement se fait après une durée de fonctionnement de l'étape d) qui se situe entre 70 et 100 %, de préférence entre 85 et 100 % de la durée maximale de fonctionnement t_{Dmax} de l'étape d) pour atteindre la valeur maximalement tolérable, telle que définie ci-dessous .

Au cours de l'étape d') du procédé, qui est effectuée simultanément à l'étape d), le catalyseur désactivé des au moins deux lits catalytiques A1 et A2 du réacteur principal R1 est régénéré et/ou remplacé par du catalyseur frais.

Selon une variante, on peut effectuer une étape a'), simultanée à l'étape a), durant laquelle le catalyseur désactivé durant l'étape d) du ou des lits catalytiques du réacteur de garde est régénéré et/ou remplacé par du catalyseur frais.

Le cycle recommence ensuite de nouveau. Les opérations sur les vannes de l'unité permettant le fonctionnement du réacteur principal R1 et du réacteur de garde B sont reportées au tableau suivant :

| Étape Cycle | | V1 | V2 | V4 | V5 | V8 |
|---|---|---|---|---|---|---|
| a | A1 + A2 | Fermé | Ouvert | Fermé | Fermé | Ouvert |
| b | B + A2 | Ouvert | Fermé | Fermé | Ouvert | Ouvert |
| c | A1 + A2 | Fermé | Ouvert | Fermé | Fermé | Ouvert |
| d | B | Ouvert | Fermé | Ouvert | Fermé | Fermé |
| a | A1 + A2 | Fermé | Ouvert | Fermé | Fermé | Ouvert |

### Taille du réacteur de garde

Afin de bénéficier des avantages du procédé selon l'invention, il est important que la taille du réacteur de garde B contenant au moins un lit catalytique soit de taille plus petite que le réacteur principal.

Plus particulièrement, le volume total du catalyseur contenu dans le ou les lits catalytiques du réacteur de garde est au maximum 60%, de préférence au maximum 50%, et de manière particulièrement préférée au maximum 40% du volume total du catalyseur contenu dans les lits catalytiques du réacteur principal.

Le réacteur de garde B peut contenir un ou plusieurs lits catalytiques. Lorsqu'il contient plusieurs lits catalytiques, il peut être muni ou non d'une boîte de trempe (quench selon le terme anglo-saxon) entre deux lits catalytiques.

Cette boîte de quench peut être alimentée avec un quench liquide, tel que la charge fraiche ou le recycle, et/ou avec un quench gazeux tel que la phase gazeuse contenant de l'hydrogène. Son débit de diluant (le plus souvent le recycle) peut être équivalent ou bien supérieur par rapport au débit de la charge, ce qui peut permettre de réduire l'exotherme.

### Temps de désactivation

Dans le cadre de l'invention, le ou les catalyseurs contenus dans le ou les lits catalytiques du réacteur principal ou du réacteur de garde, notamment le premier lit catalytique dans le sens de la circulation de la charge, se désactivent progressivement. Lorsque le(s) catalyseur(s) est/sont désactivé(s) ou partiellement désactivé(s), on procède au basculement d'une étape à une autre étape.

La durée de fonctionnement pour chacune des étapes a), b), c) et d) se définit par rapport à une durée maximale de fonctionnement pour chaque étape, elle-même définie par rapport à une valeur maximalement tolérable d'une température ou d'une sélectivité. La valeur maximalement tolérable est définie au préalable par l'homme du métier et dépend notamment de la nature de la charge à traiter, mais aussi des conditions opératoires et des catalyseurs choisis. Lorsque la valeur maximalement tolérable est atteinte, il faut procéder à un basculement d'étape, c'est-à-dire à un court-circuitage d'un lit catalytique, ou à la déconnection d'un réacteur et/ou à la connexion d'un autre réacteur.

Plus particulièrement, pour une charge C3, pour laquelle on n'observe pas ou peu de changement de température durant un cycle, la valeur maximalement tolérable est la sélectivité requise par les spécifications (généralement correspondant à l'atteinte d'une égalité de composition en propylène entre l'entrée et la sortie du réacteur) en fin de cycle. En effet, lorsque le catalyseur se désactive, on observe une baisse progressive en sélectivité (formation de plus en plus forte de propane, et baisse de la pureté en propylène en sortie de réacteur).

Lorsque la charge est une charge C4, la valeur maximalement tolérable est une température qui se situe à 160°C, préférentiellement 120°C en sortie de réacteur en fin de cycle. En effet, au-dessus de cette température on observe une baisse de la sélectivité de la réaction d'hydrogénation (formation d'oligomères). Cette fourchette de températures est également celle maximalement tolérable pour une charge C5, toujours du fait d'une baisse de la sélectivité.

Lorsque la charge est une charge d'essence de pyrolyse, la valeur maximalement tolérable est une température qui se situe à environ 200°C, préférentiellement 180°C en sortie de réacteur en fin de cycle. En effet, au-dessus de cette température on observe une baisse de la sélectivité (hydrogénation d'aromatiques) avec en plus de forts risques d'emballement thermique.

La durée maximale de fonctionnement tₘₐₓ pour chacune des étapes a), b) c) et d) se définit ainsi comme le temps pour lequel la valeur maximalement tolérable de la température ou de la sélectivité est atteinte. Par exemple, dans le cas d'une charge d'essence de pyrolyse, la durée maximale de fonctionnement t_{Amax} pour l'étape a) est le temps nécessaire pour atteindre la valeur maximalement tolérable de 180°C et la durée maximale de fonctionnement t_{Bmax} pour l'étape b) est le temps nécessaire pour atteindre la valeur maximalement tolérable de 180°C et ainsi de suite. Les durées maximales de fonctionnement de chacune des étapes peuvent être différentes puisque dépendantes de la configuration de l'installation et de la désactivation du catalyseur en début d'étape.

La durée de fonctionnement de chacune des étapes a), b), c) et d) est ainsi respectivement :
- pour l'étape a) : entre 10 et 70 %, de préférence entre 20 et 60 % de t_{Amax}
- pour l'étape b) : entre 40 et 100 %, de préférence entre 70 et 100 % de t_{Bmax}
- pour l'étape c) : entre 90 et 100 %, de préférence 100 % de t_{Cmax}
- pour l'étape d) : entre 70 et 100 %, de préférence entre 85 et 100 % de t_{Dmax}.

Ainsi, par exemple, le basculement de l'étape a) durant laquelle la charge traverse les lits catalytiques A1 et A2 du réacteur principal à l'étape b) durant laquelle la charge traverse le réacteur de garde B puis le lit catalytique A2 du réacteur principal se fait après une durée de fonctionnement de l'étape a) qui se situe entre 10 et 70 %, de préférence entre 20 et 60 % de la durée maximale de fonctionnement t_{Amax} pour atteindre la valeur maximalement tolérable durant cette étape a) (par exemple 180°C pour une charge d'essence de pyrolyse). En effet, le basculement de l'étape a) vers l'étape b) est effectué lorsque le premier lit catalytique A1 est partiellement désactivé ce qui est exprimé par une durée de fonctionnement de l'étape a) qui se situe entre 10 et 70 % de t_{Amax}.

Dans une forme préférée de réalisation, les lits catalytiques contenus dans le réacteur principal peuvent être de volumes différents ou identiques avec néanmoins comme condition préférée que le volume du dernier lit soit plus grand que chaque volume des autres lits. De préférence, les lits catalytiques dans le réacteur principal sont de volumes croissants dans le sens de l'écoulement. En effet, puisque la montée en températures due à l'exothermie des réactions d'hydrogénation se fait principalement sur le premier lit catalytique, il est avantageux de minimiser le volume de ce premier lit.

### Conditions d'hydrogénation

Dans le cadre du procédé selon l'invention, la charge polyinsaturée est mise en contact avec une phase gazeuse comprenant de l'hydrogène en présence d'un catalyseur d'hydrogénation, dans des conditions, notamment de température, de pression, et de vitesse volumique horaire (VVH), permettant l'hydrogénation.

En particulier, le procédé d'hydrogénation sélective est avantageusement effectué sous pression, en phase mixte gazeuse/liquide, en présence d'hydrogène.

Le procédé d'hydrogénation sélective selon l'invention est, de préférence, mis en oeuvre dans chaque réacteur à une température allant de 0°C à 200°C.

La pression dans chaque réacteur est de préférence comprise entre 1 et 6,5 MPa, plus préférentiellement comprise entre 1,5 et 5 MPa et encore plus préférentiellement comprise entre 1,5 et 3,5 MPa.

La vitesse volumique horaire globale (VVH), définie comme le rapport du débit volumique de la charge fraîche à 15°C sur le volume total de catalyseur présent dans l'ensemble des réacteurs mis en oeuvre, est généralement de 1 h⁻¹ à 100 h⁻¹, de préférence de 1 h⁻¹ à 50 h⁻¹.

Lorsque la charge est une charge C3, le procédé d'hydrogénation sélective est habituellement effectuée comme un procédé en phase gazeuse ou en phase mixte gazeuse/liquide avec une vitesse volumique horaire globale de 5 h⁻¹ à 30 h⁻¹, à une température de 0°C à 70°C, et à une pression comprise entre 1,5 et 5 MPa. Le taux de recyclage, défini comme le rapport du débit massique de recycle sur le débit massique de charge entrant dans le réacteur, peut notamment être entre 0 et 5.

Lorsque la charge est une charge C4, le procédé d'hydrogénation sélective est habituellement effectuée comme un procédé en phase mixte gazeuse/liquide avec une vitesse volumique horaire globale de 2 h⁻¹ à 15 h⁻¹, à une température de 30°C à 160°C, et à une pression comprise entre 1,5 et 5 MPa. Le taux de recyclage, défini comme le rapport du débit massique de recycle sur le débit massique de charge entrant dans le réacteur, peut notamment être entre 0 et 30. Lorsque la charge est une charge C5, le procédé d'hydrogénation sélective est habituellement effectuée comme un procédé en phase mixte gazeuse/liquide avec une vitesse volumique horaire globale de 1 h⁻¹ à 10 h⁻¹, à une température de 30°C à 160°C, et à une pression comprise entre 1,5 et 5 MPa. Le taux de recyclage, défini comme le rapport du débit massique de recycle sur le débit massique de charge entrant dans le réacteur, peut notamment être entre 0 et 15, de préférence entre 0,5 et 15.

De manière très préférée, on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 1 et 2, la température est généralement comprise entre 40°C et 200°C, de préférence entre 50 et 180°C, la vitesse volumique horaire (V.V.H.) est comprise généralement entre 1 h⁻¹ et 6 h⁻¹ et la pression est généralement comprise entre 2 MPa et 6 MPa. Le débit d'hydrogène est ajusté afin d'en disposer en quantité suffisante pour hydrogéner théoriquement l'ensemble des composés polyinsaturés et de maintenir un excès d'hydrogène en sortie de réacteur. Le taux de recyclage, défini comme le rapport du débit massique de recycle sur le débit massique de charge entrant dans le réacteur, peut notamment être entre 0 et 5, de préférence entre 0.5 et 5.

Les conditions opératoires peuvent être identiques ou différentes dans le réacteur principal et le réacteur de garde durant les étapes a), b), c) et d), de préférence ils sont identiques, notamment durant les étapes a) et b) et c).

De même, les conditions opératoires dans le réacteur principal peuvent être identiques ou différentes durant les étapes a) et c).

Afin d'assurer une hydrogénation sélective dans le volume de catalyseur réduit dans le ou les lits catalytiques du réacteur B durant l'étape d) lorsque la charge ne traverse que le réacteur de garde et que le catalyseur dans le réacteur principal est remplacé et/ou régénéré, les conditions d'opérations de l'étape d) sont de préférence plus sévères que les conditions d'opérations utilisées en début de l'étape a) (ou b) et c)) impliquant une étape d'hydrogénation sélective comme définies ci-dessus.

### Charge

Ladite charge d'hydrocarbures comprend au moins une molécule polyinsaturée comprenant au moins 3 atomes de carbone, et est sélectionnée dans le groupe constitué par une coupe C3 de vapocraquage, une coupe C4 de vapocraquage, une coupe C5 de vapocraquage et une essence de pyrolyse et de leur mélange. Plus précisément, lesdits hydrocarbures polyinsaturés présents dans ladite charge traitée sont en particulier des composés comportant au moins une fonction acétylénique (c'est-à-dire au moins une triple liaison) et/ou au moins une fonction diénique (c'est-à-dire au moins deux double liaisons) et/ou au moins une fonction alcényl-aromatique (c'est-à-dire au moins un cycle aromatique contenant au moins un ligand oléfinique). En particulier, ladite charge d'hydrocarbures polyinsaturés peut comprendre au moins un type de composé contenant à la fois une fonction acétylène et une fonction diénique par molécule. La charge d'hydrocarbures est sélectionnée dans le groupe constitué par une coupe C3 de vapocraquage, une coupe C4 de vapocraquage, une coupe C5 de vapocraquage et une essence de vapocraquage encore appelée essence de pyrolyse. La charge essence de pyrolyse peut en plus comprendre des alcénylaromatiques.

La coupe C3 de vapocraquage, avantageusement utilisée pour la mise en oeuvre du procédé d'hydrogénation sélective selon l'invention, comprend généralement du propylène, du propadiène, du méthylacétylène, et du propane. La coupe C3 présente par exemple la composition moyenne suivante : de l'ordre de 90% poids de propylène, de l'ordre de 2 à 8% poids de propadiène et de méthylacétylène, le reste étant essentiellement du propane. Dans certaines coupes C3, entre 0,1 et 2% poids de composés en C2 et de composés en C4 peuvent aussi être présents. La coupe C4 de vapocraquage, avantageusement utilisée pour la mise en oeuvre du procédé d'hydrogénation sélective selon l'invention, comprend généralement du butane, du butène, du butadiène, du vinylacetylène et du butyne. La coupe C4 présente par exemple la composition massique moyenne suivante : 1 % poids de butane, 46,5% poids de butène, 51% poids de butadiène, 1,3% poids de vinylacetylène (VAC) et 0,2% poids de butyne. Dans certaines coupes C4, entre 0,1 et 2% poids de composés en C3 et de composés en C5 peuvent aussi être présents.

La coupe C5 de vapocraquage, avantageusement utilisée pour la mise en oeuvre du procédé d'hydrogénation sélective selon l'invention, comprend généralement de pentanes, des pentènes et des pentadiènes. La coupe C5 présente par exemple la composition suivante : 21% poids de pentanes, 45% poids de pentènes, 34% poids de pentadiènes.

L'essence de vapocraquage ou essence de pyrolyse, avantageusement utilisée pour la mise en oeuvre du procédé d'hydrogénation sélective selon l'invention, correspond à une coupe hydrocarbonée dont la température d'ébullition est généralement comprise entre 0°C et 250°C, de préférence entre 10°C et 220°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition en % poids suivante: 5 à 15 % poids de paraffines, 50 à 65 % poids de composés aromatiques, 5 à 15% poids de mono-oléfines, 15 à 25 % poids de dioléfines, 2 à 8 % poids de composés alkénylaromatiques et de 20 à 300 ppm poids de soufre (partie part million), voire jusqu'à 2000 ppm de soufre pour certaines charges difficiles, l'ensemble des composés formant 100%.

De manière préférée, la charge d'hydrocarbures polyinsaturés traitée conformément au procédé d'hydrogénation sélective selon l'invention est une essence de vapocraquage.

Lorsque ladite charge est une coupe C3, le procédé d'hydrogénation sélective selon l'invention vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective selon l'invention vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants.

### Phase gazeuse

Dans le cadre du procédé selon l'invention, la charge d'hydrocarbures est mise en contact avec une phase gazeuse comprenant de l'hydrogène.

La phase gazeuse est souvent composée d'un mélange d'hydrogène et d'au moins un autre gaz, inerte pour la réaction selon le procédé de purification utilisé. Cet autre gaz peut, par exemple, être choisi dans le groupe formé par le méthane, l'éthane, le propane, le butane, l'azote, l'argon, le monoxyde de carbone (quelques ppm) et le dioxyde de carbone. Cet autre gaz est préférentiellement du méthane ou du propane, et est plus préférentiellement exempt de monoxyde de carbone. La quantité d'hydrogène est de préférence faiblement en excès par rapport à la valeur stoechiométrique, permettant l'hydrogénation sélective des composés polyinsaturés présents dans la charge d'hydrocarbures. Dans ce mode de réalisation, l'excès d'hydrogène est compris généralement entre 1 et 50% en poids, de préférence entre 1 et 30% en poids.

La proportion d'hydrogène dans la phase gazeuse est notamment de 60 % à 100 % en poids, et le plus souvent de 80 % à 99,99 % en poids, le complément à 100 % étant l'un ou plusieurs des gaz inertes précédemment cités.

Selon un mode particulièrement préféré de l'invention, la phase gazeuse est constituée d'hydrogène à 100% en poids.

La phase gazeuse comprenant l'hydrogène est, de préférence, au moins introduite en tête du premier réacteur traversé par la charge, et peut avantageusement être également introduite en tête de chaque lit catalytique.

En étageant ainsi l'introduction de la phase gazeuse comprenant de l'hydrogène entre les différents réacteurs, il est possible d'introduire des quantités moindres d'hydrogène en tête de chaque réacteur, ce qui limite le risque de réactions secondaires dans chaque réacteur. En outre, l'introduction d'une phase gazeuse comprenant de l'hydrogène en tête de chaque réacteur et à une température proche de l'ambiante (environ 20°C), permet d'abaisser la température de la charge introduite dans le réacteur aval (la réaction d'hydrogénation étant exothermique), limitant ainsi la vaporisation des charges oléfiniques ce qui favorise une meilleure sélectivité. La conjonction d'une faible introduction d'hydrogène et d'une charge oléfinique maintenue liquide entraine une meilleure solubilisation de l'hydrogène dans la charge, de sorte que le réacteur s'approche d'un régime monophasique liquide. Ces régimes proches du régime monophasique permettent d'améliorer encore la sélectivité des réactions d'hydrogénation.

La phase gazeuse comprenant de l'hydrogène peut aussi être introduite pour partie en mélange avec la charge d'hydrocarbures avant le premier lit de catalyseur, et pour partie avant le ou les lits suivants contenus dans ledit réacteur (aussi appelé « quench » selon la terminologie anglo-saxonne) afin de limiter le gradient de température des réactions exothermiques d'hydrogénation dans le réacteur.

### Catalyseur d'hydrogénation

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

Le catalyseur utilisé dans le procédé selon l'invention est un catalyseur connu de l'homme du métier pour un procédé d'hydrogénation sélective. Il peut préférentiellement comprendre au moins un métal du groupe VIII, plus préférentiellement le palladium ou le nickel.

Le métal du groupe VIII, de préférence le palladium peut, de préférence, être déposé en croute à la périphérie du support (billes, extrudés). La répartition en croute est bien connue de l'homme du métier et permet une meilleure sélectivité du catalyseur en ce sens que les molécules polyinsaturées sont bien converties en monooléfines, mais les monooléfines ne sont pas hydrogénées en alcanes. Lorsque le métal du groupe VIII est le palladium, la teneur en palladium est comprise entre 0,01 et 2 % poids de la masse du catalyseur, de préférence de 0,03 et 0,8 % en poids.

Lorsque le métal du groupe VIII est le nickel, la teneur en nickel est comprise entre 1 et 50 % poids de la masse du catalyseur, de préférence entre 5 et 40 % poids et plus préférentiellement entre 7 et 30 % poids.

Les valeurs « % poids » se basent sur la forme élémentaire du métal du groupe VIII.

Le catalyseur comprend notamment un support poreux formé d'au moins un oxyde simple choisi parmi l'alumine (Al₂O₃), la silice (SiO₂), l'oxyde de titane (TiO₂), la cérine (CeO₂) et la zircone (ZrO₂). De manière préférée, ledit support est choisi parmi les alumines, les silices et les silices-alumines. De manière particulièrement préférée, le support poreux est une alumine.

Le support poreux peut notamment se présenter sous forme de billes, d'extrudés, par exemple sous forme de trilobes ou de quadrilobes, de pastilles, ou d'agglomérats irréguliers et non sphériques dont la forme spécifique peut résulter d'une étape de concassage.

De manière très avantageuse, le support se présente sous forme de billes ou d'extrudés.

De préférence, le catalyseur utilisé peut comprendre, en outre, au moins un agent dopant, appartement à la colonne IB du tableau périodique, qui peut être de préférence choisi dans le groupe formé par l'or, l'argent et le cuivre, et plus préférentiellement l'argent. Il peut également comprendre de l'étain.

De préférence, les catalyseurs d'hydrogénation sélective comprennent, en outre, au moins un métal sélectionné dans le groupe constitué par les alcalins et les alcalino-terreux.

Préalablement à une utilisation dans un procédé d'hydrogénation sélective, les catalyseurs d'hydrogénation sélective subissent généralement au moins un traitement réducteur, éventuellement suivi d'une passivation, généralement au soufre.

Chaque lit catalytique contient au moins une couche catalytique contenant un ou plusieurs catalyseurs, éventuellement complété par au moins une couche inerte en tête de lit catalytique. Les catalyseurs utilisés dans le ou les lit(s) catalytique(s) des réacteurs peuvent être identiques ou différents. Le catalyseur utilisé dans le réacteur de garde peut être identique ou non au catalyseur utilisé dans le réacteur principal.

### Régénération / Réactivation

Lorsque le catalyseur d'un des réacteurs est désactivé, il est régénéré et/ou réjuvéné.

La régénération du catalyseur peut notamment être effectuée à une température allant de 200 à 480°C, avec une montée progressive par rampes de la température, sous azote, et avec des ajouts successifs de vapeur d'eau (steam stripping selon la terminologie anglo-saxonne) et d'oxygène (combustion). Le catalyseur est ensuite réactivé sous hydrogène, et éventuellement avec ajout de molécules soufrées, pour reprendre son état initial.

La réjuvénation du catalyseur (hot hydrogen stripping selon la terminologie anglo-saxonne) peut notamment être effectuée à une température allant de 200 à 450°C, avec une montée progressive par rampes de la température, sous azote et hydrogène.

Une telle procédure peut notamment permettre de limiter le frittage des particules métalliques et la dégradation du support.

Suivant les étapes considérées a) à d), un choix entre régénération et réjuvénation pourra être effectué par l'homme du métier. Par exemple, lors de l'étape d'), l'homme du métier choisira préférentiellement une régénération du catalyseur.

On entend dans ce texte par un « catalyseur régénéré » ou par une « étape dans laquelle le catalyseur est régénéré », un catalyseur régénéré et/ou réjuvéné ou une étape dans laquelle le catalyseur est régénéré et/ou réjuvéné.

### Recyclage

Selon un mode de réalisation préféré de l'invention, une partie de l'effluent gaz/liquide issu du réacteur principal ou du réacteur de garde peut être renvoyée (i.e. recyclée) en mélange avec la charge à hydrotraiter.

Selon un autre mode de réalisation préféré de l'invention, une partie de l'effluent gaz/liquide issu du réacteur principal ou du réacteur de garde peut être renvoyée (i.e. recyclée) à l'entrée dudit réacteur et/ou d'un réacteur ou lit catalytique se situant en amont, de préférence d'un réacteur ou lit catalytique se situant immédiatement en amont.

L'objectif de ces recycles est de diluer la charge en entrée du réacteur, pour limiter la montée en température et donc la formation d'oligomères (ou gommes). L'effluent issu du réacteur principal ou du réacteur de garde peut subir avant d'être recyclé une séparation de la phase gazeuse (hydrogène non réagit) afin de ne recycler que la phase liquide.

Lorsque le procédé selon l'invention met en oeuvre un recyclage, la phase gazeuse comprenant l'hydrogène peut être introduite avant et/ou après l'introduction du recycle.

Selon un autre mode de réalisation, une trempe (quench selon le terme anglo-saxon) peut être introduite entre deux lits catalytiques dans le réacteur principal ou le réacteur de garde afin d'abaisser la température dans le lit catalytique en aval. Ce quench peut être un quench liquide, tel que la charge fraiche ou le recycle, et/ou un quench gazeux tel que la phase gazeuse contenant de l'hydrogène.

Selon encore un autre mode de réalisation, le procédé selon l'invention peut être opéré à courant descendant (downflow selon le terme anglo-saxon) ou ascendant (upflow selon le terme anglo-saxon). Lorsqu'il est opéré à courant ascendant, le lit catalytique A1 du réacteur principal se trouve ainsi en-dessous du lit catalytique A2.

### Echangeur de chaleur (refroidisseur)

Selon un mode préféré de réalisation, le procédé selon l'invention peut notamment mettre en oeuvre un ou plusieurs échangeur(s) de chaleur (refroidisseur) entre chaque réacteur de manière à refroidir l'effluent du réacteur situé immédiatement en amont, et notamment entre le réacteur de garde et le réacteur principal. La température de l'effluent est ainsi abaissée de manière à liquéfier les oléfines vaporisées dans le réacteur de garde B. Cet échangeur de chaleur intermédiaire permet donc de contrôler l'exothermie.

Lorsque le procédé selon l'invention met en oeuvre un ou plusieurs échangeur(s) de chaleur, la phase gazeuse comprenant l'hydrogène peut être introduite en amont et/ou en aval dudit échangeur.

Selon un autre mode de réalisation, chacun des recycles peut être refroidi par un échangeur de chaleur avant d'être introduit dans un réacteur.

### Autres modes de réalisation

D'autres modes de réalisation du procédé de l'invention peuvent encore être envisagés afin de traiter des charges plus difficiles ou de soulager un lit catalytique lors des différentes étapes, tels que illustrés sur la figure 2.

Selon un mode de réalisation, le réacteur de garde peut être utilisé en amont du réacteur principal durant l'étape a) afin de traiter des charges plus difficiles. La charge est ainsi introduite dans le réacteur de garde B via la vanne V1, puis traverse via la vanne V6 les deux lits catalytiques A1 et A2 du réacteur principal. Dans ce cas, la vanne V2 est fermée lors de l'étape a).

Selon un autre mode de réalisation et afin de soulager le premier lit catalytique A1 lors de l'étape a) et/ou de traiter des charges plus difficiles, une partie de la charge peut être introduite dans le réacteur de garde via la vanne V1 en plus de son introduction dans le premier lit catalytique via la vanne V2 lors de l'étape a). La charge est ainsi introduite en parallèle dans le réacteur de garde et dans le premier lit catalytique du réacteur de garde, les vannes V1, V2 et V5 sont ouvertes, la vanne V4 est fermée.

Selon un autre mode de réalisation et afin d'utiliser pleinement les capacités catalytiques du deuxième lit catalytique A2 du réacteur principal lors de l'étape a), une partie de la charge peut être introduite dans le deuxième lit catalytique A2 via la vanne V3 en plus de son introduction dans le premier lit catalytique A1 via la vanne V2 lors de l'étape a). La charge est ainsi introduite en parallèle dans les deux lits catalytiques A1 et A2 du réacteur principal. Les vannes V2 et V3 sont ouvertes. Selon ce mode de réalisation, une partie de l'effluent du réacteur principal peut être recyclée dans le réacteur de garde B via la vanne V7 afin de compenser le court-circuitage du lit catalytique A1 pour une partie de la charge.

Selon encore un autre mode de réalisation, lorsque la charge traverse successivement le réacteur de garde B puis le deuxième lit catalytique du réacteur principal lors de l'étape b), une partie de la charge peut être également introduite sur le premier lit catalytique partiellement désactivé A1 du réacteur principal. La charge est ainsi introduite en parallèle dans le réacteur de garde et dans le premier lit catalytique du réacteur de garde, les vannes V1, V2 et V5 sont ouvertes, la vanne V4 est fermée.

Selon un autre mode de réalisation, la sélectivité de l'hydrogénation peut être abaissée, en ayant pour objectif de saturer les oléfines jusqu'à ce qu'il en reste entre 10 et 15%.

### Exemples

### Exemple 1 selon l'art antérieur

Une charge d'essence de pyrolyse « PyGas » ayant une valeur MAV de 210 (MAV pour Maleic Anhydride Value selon la terminologie anglo-saxonne, mesure en teneur en dioléfines) et un indice de brome de 81 (mesure en teneur en oléfines), contenant 3% de styrène (et 8% de composés styréniques C9+), a été traitée par un procédé d'hydrogénation tel qu'illustré à la figure 1, dans les conditions opératoires suivantes :
- Débit de charge : 17 t/h
- Composition de la phase gazeuse comprenant de l'hydrogène: 95% H₂, 5% CH₄
- Débit total d'hydrogène : 0,4 t/h (H₂+CH₄)
- VVH, définie comme le rapport du débit volumique de charge fraîche à 15°C au volume de catalyseur : 1,5h⁻¹
- Volume de catalyseur de 17 m³ dans un réacteur de 1000 mm diamètre (1° réacteur principal, catalyseur actif)
- Réserve de catalyseur de 17 m³ dans un réacteur de 1000 mm diamètre (2° réacteur principal, catalyseur inactif)
- Débit de recycle : 30 t/h
- Pression absolue en entrée réacteur : 3 MPa (30 bars)
- Température en entrée réacteur : 60°C

L'objectif dans cet exemple est d'abaisser la teneur en styrène (et a fortiori la plupart des dioléfines qui sont plus faciles à hydrogéner) à 0,5 %pds en sortie du réacteur.

Dans ce mode de réalisation, un seul réacteur est utilisé pour l'hydrogénation. 100% de la conversion souhaitée en styrène doit donc être effectuée dans ce réacteur.

Le procédé selon le présent exemple permet donc d'hydrogéner la charge d'essence de pyrolyse sur une durée de cycle estimée de 6 mois, ce qui permet avec le 2° réacteur principal d'utiliser l'intégralité du volume catalytique sur une durée de 12 mois.

| Étape Cycle | | Durée |
|---|---|---|
| a | 1° Réacteur principal | 6 mois |
| b | 2° Réacteur principal | 6 mois |
| a | 1° Réacteur principal | 6 mois |

### Exemple 2 selon l'invention

La même charge oléfinique que celle traitée dans l'exemple 1 (comparatif) a été traitée par un procédé d'hydrogénation selon l'invention, comprenant deux réacteurs en série et parallèle, avec ou sans boîte de quench, tel qu'illustré à la figure 2. Une masse de catalyseur inférieure de 15%vol (28 m³ au lieu de 34 m³) à celle utilisée dans les deux réacteurs de l'exemple 1 a été répartie à 70% dans le réacteur principal (30% dans le premier lit, 40% dans le second), et à 30% dans le réacteur de garde. Le procédé de l'exemple 1 (comparatif) et celui de l'exemple 2 (selon l'invention) sont comparés à iso-débit (30 t/h) et qualité de charge et de gaz (0,4 t/h H₂ à 95% CH₄ 5%). Les conditions opératoires sont les suivantes :
- la VVH du réacteur principal, définie comme le rapport du débit volumique de charge fraîche à 15°C au volume de catalyseur du réacteur principal : 1,5 h⁻¹
- Volume de catalyseur de 20 m³ dans un réacteur de 1000 mm diamètre (réacteur principal, catalyseur actif)
- Volume de catalyseur de 8 m³ dans un réacteur de 1000 mm diamètre (réacteur de garde, catalyseur actif)
- Diamètre des réacteurs de 1000 mm
- Débit de recycle : 30 t/h
- Pression absolue dans les réacteurs : 3 MPa (30 bars)
- Température d'entrée dans le 1^{er} réacteur : 60°C

L'objectif dans cet exemple est de maintenir la conversion en styrène à 0,5 %pds en sortie du procédé, sur une durée plus longue que le procédé de référence, grâce à une réorganisation des lits catalytiques et à un séquençage optimal de ceux-ci, tout en réduisant le volume total de catalyseur (comptabilisé comme la somme des volumes du réacteur principal et du réacteur de garde). Le tableau suivant reprend les étapes du cycle d'utilisation de l'intégralité du catalyseur.

| Étape Cycle | | Durée | V1 | V2 | V4 | V5 | V8 | Régénération |
|---|---|---|---|---|---|---|---|---|
| a | A1 + A2 | 3,5 mois | Fermé | Ouvert | Fermé | Fermé | Ouvert | B |
| b | B + A2 | 3,5 mois | Ouvert | Fermé | Fermé | Ouvert | Ouvert | |
| c | A1 + A2 | 3,5 mois | Fermé | Ouvert | Fermé | Fermé | Ouvert | B |
| d | B | 3 mois | Ouvert | Fermé | Ouvert | Fermé | Fermé | A1+A2 |
| a | A1 + A2 | 3,5 mois | Fermé | Ouvert | Fermé | Fermé | Ouvert | B |

Le tableau suivant reprend pour chaque étape les durées maximales de fonctionnement tₘₐₓ, les durées de fonctionnement (en pourcentage), ainsi que les durées réelles :

| Étape Cycle | | tₘₐₓ | Durée de fonctionnement | Durée |
|---|---|---|---|---|
| a | A1 + A2 | 7 mois | 50% | 3,5 mois |
| b | B + A2 | 7 mois | 50% | 3,5 mois |
| c | A1 + A2 | 3,5 mois | 100% | 3,5 mois |
| d | B | 3 mois | 100% | 3 mois |
| a | A1 + A2 | 7 mois | 50% | 3,5 mois |

Lors des étapes a) et c), le catalyseur du réacteur de garde B est régénéré, et lors de l'étape d), le catalyseur du réacteur principal R1 (A1+A2) est régénéré. La durée de l'étape a) est déterminée dans cet exemple par rapport à la durée maximale de fonctionnement t_{Amax} qui est de 7 mois par rapport à une température de 180 °C en fin de cycle. En effet, les volumes des lits A1 et A2 étant supérieurs de 20% à ceux de l'exemple comparatif, il a été évalué que la désactivation suivait un rythme inférieur qui permet d'atteindre une durée de 3,5 mois pour l'étape a). La durée de l'étape b) est similaire à celle de l'étape a) car le volume de catalyseur du réacteur de garde B est ici égal à celui de A1, et en prenant en compte la désactivation légère du lit A2, on obtient encore une durée d'étape évaluée à 3,5 mois.

La durée de l'étape c) est impactée de façon négligeable par la désactivation de A2 lors de l'étape b), et on évalue la durée des étapes combinées a) et c) à la durée de cycle classique de l'exemple de référence (exemple 1), ce qui correspond pour ce volume de catalyseur à 7 mois, et donc une durée de l'étape c) de 3,5 mois.

Lors de l'étape c), le catalyseur du réacteur B a été régénéré ou remplacé, et l'étape d) démarre sur catalyseur à activité maximale. Dans ces conditions, une température de démarrage de 63°C d'obtenir la conversion attendue (0,5 %pds de styrène en sortie) pour un volume de catalyseur moindre. Afin d'améliorer le contrôle de l'exotherme, il est possible de modifier le taux de diluent ou de munir B d'une boîte de quench. La conversion est atteinte pour une température d'effluent limitant la durée de l'étape d) entre 3 et 3,5 mois.

Le procédé selon l'invention (exemple 2) permet donc d'augmenter la durée de cycle d'utilisation totale du catalyseur procédé de 12 à 15% (13,5 mois au lieu de 12) tout en réduisant le volume de catalyseur de 15%vol, et la taille des équipements associés.

## Revendications

1. Procédé d'hydrogénation sélective d'une charge d'hydrocarbures contenant des molécules polyinsaturées comprenant au moins 3 atomes de carbone et sélectionnée dans le groupe constitué par une coupe C3 de vapocraquage, une coupe C4 de vapocraquage, une coupe C5 de vapocraquage et une essence de pyrolyse et de leur mélange, dans lequel on fait passer, dans des conditions d'hydrogénation, ladite charge et une phase gazeuse comprenant de l'hydrogène sur un catalyseur d'hydrogénation, dans un seul réacteur principal à lit fixe contenant au moins deux lits catalytiques, et un réacteur de garde à lit fixe de taille plus petite contenant au moins un lit catalytique, lesdits réacteurs étant disposés en série pour être utilisés de façon cyclique en répétant successivement des étapes a), b), c), c'), d) et d') définies ci-après :
- une étape a) durant laquelle la charge traverse successivement tous les lits catalytiques du réacteur principal,
- lorsque le premier lit catalytique du réacteur principal commence à se désactiver, une étape b) durant laquelle la charge est introduite dans le réacteur de garde puis, en court-circuitant le premier lit catalytique partiellement désactivé du réacteur principal, dans le prochain lit catalytique non désactivé dudit réacteur principal situé immédiatement en aval par rapport au sens de circulation de la charge,
- une étape c) durant laquelle la charge traverse uniquement et successivement tous les lits catalytiques du réacteur principal,
- une étape c'), simultanée à l'étape c), durant laquelle le catalyseur désactivé du ou des lits catalytiques du réacteur de garde est régénéré et/ou remplacé par du catalyseur frais,
- une étape d) durant laquelle la charge ne traverse que le réacteur de garde,
- une étape d'), simultanée à l'étape d), durant laquelle le catalyseur désactivé des au moins deux lits catalytiques du réacteur principal est régénéré et/ou remplacé par du catalyseur frais ;
procédé dans lequel la durée de fonctionnement pour chacune des étapes a), b), c) et d), défini par rapport à une durée maximale de fonctionnement tₘₐₓ pour chaque étape qui est le temps pour atteindre une valeur maximalement tolérable d'une température ou d'une sélectivité pour une charge donnée, est respectivement :
• pour l'étape a) : entre 10 et 70 % de t_{Amax}
• pour l'étape b) : entre 40 et 100 % de t_{Bmax}
• pour l'étape c) : entre 90 et 100 % de t_{Cmax}
• pour l'étape d) : entre 70 et 100 % de t_{Dmax} ;
et étant entendu que
• la valeur maximalement tolérable d'une charge C3 est la sélectivité correspondant à l'atteinte d'une égalité de composition en propylène entre l'entrée et la sortie du réacteur en fin de cycle ;
• la valeur maximalement tolérable d'une charge C4 est une température maximale de 160 °C;
• la valeur maximalement tolérable d'une charge C5 est une température maximale de 160 °C;
• la valeur maximalement tolérable d'une charge d'essence de pyrolyse est une température maximale de 200°C.

2. Procédé selon la revendication 1, dans lequel le volume total du catalyseur contenu dans le ou les lits catalytiques du réacteur de garde est au maximum 60 % du volume total du catalyseur contenu dans les lits catalytiques du réacteur principal.

3. Procédé selon la revendication 1, dans lequel on effectue une étape a'), simultanée à l'étape a), durant laquelle le catalyseur désactivé durant l'étape d) du ou des lits catalytiques du réacteur de garde est régénéré et/ou remplacé par du catalyseur frais.

4. Procédé selon l'une des revendications précédentes, dans lequel chaque réacteur est mis en oeuvre à une température allant de 0°C à 200°C, à une pression comprise entre 1 et 6,5 MPa et à une vitesse volumique horaire globale, définie comme le rapport du débit volumique de la charge fraîche à 15°C sur le volume total de catalyseur présent dans l'ensemble des réacteurs mis en oeuvre, comprise entre 1 h⁻¹ et 100 h⁻¹.

5. Procédé selon l'une des revendications précédentes, dans lequel une partie de l'effluent issue du réacteur principal ou du réacteur de garde est recyclée en mélange avec la charge à hydrogéner.

6. Procédé selon l'une des revendications précédentes, dans lequel les conditions opératoires d'hydrogénation durant les étapes a) b) c) et d) sont identiques.

7. Procédé selon l'une des revendications 1 à 5 dans lequel durant l'étape d) la température du réacteur de garde est augmentée et/ou le flux de la phase comprenant de l'hydrogène est augmenté, et/ou le débit de recycle dans le réacteur de garde est augmenté et/ou le débit de charge introduit dans le réacteur de garde est diminué par rapport à respectivement la température, le flux ou le(s) débit(s) utilisé(s) en début de l'étape a).

8. Procédé selon la revendication 7, dans lequel l'augmentation de la température du réacteur de garde en tête de réacteur au début de l'étape d) par rapport au début de l'étape a) est comprise entre 0,5 et 40°C.

9. Procédé selon les revendications 7 ou 8, dans lequel l'augmentation du débit de recycle au réacteur de garde au début de l'étape d) par rapport au débit de recycle en début de l'étape a) est comprise entre 0,5 et 100%.

10. Procédé selon l'une des revendications précédentes, dans lequel une trempe est introduite entre deux lits catalytiques dans le réacteur principal ou le réacteur de garde, ladite trempe pouvant être une trempe liquide et/ou une trempe gazeuse.

11. Procédé selon l'une des revendications précédentes, dans lequel on met en oeuvre un ou plusieurs échangeur(s) de chaleur entre le réacteur de garde et le réacteur principal.

12. Procédé selon l'une des revendications précédentes, dans lequel les catalyseurs d'hydrogénation utilisés sont identiques ou différents dans le ou les lits catalytiques du réacteur principal et/ou du réacteur de garde.

## Patentansprüche

1. Verfahren zur selektiven Hydrierung einer Kohlenwasserstoffcharge, die polyungesättigte Moleküle enthält, umfassend mindestens 3 Kohlenstoffatome und ausgewählt aus der Gruppe bestehend aus einer Dampfcrackfraktion C3, einer Dampfcrackfraktion C4, einer Dampfcrackfraktion C5 und einem Pyrolysebenzin und ihren Mischungen, wobei die Charge und eine Gasphase, umfassend Wasserstoff auf einem Hydrierungskatalysator unter Hydrierungsbedingungen in einen einzigen Festbett-Hauptreaktor, der mindestens zwei Katalysatorbetten enthält, und einen Schutzfestbett-Reaktor mit kleinerer Größe, der mindestens ein Katalysatorbett enthält, geführt werden, wobei die Reaktoren in Serie angeordnet sind, um zyklisch verwendet zu werden, indem aufeinanderfolgend die nachstehend definierten Schritte a), b), c), c'), d) und d') wiederholt werden:
- ein Schritt a), in dem die Charge aufeinanderfolgend alle Katalysatorbetten des Hauptreaktors durchquert,
- wenn das erste Katalysatorbett des Hauptreaktors sich zu deaktivieren beginnt, ein Schritt b), in dem die Charge in den Schutzbett-Reaktor eingebracht wird, dann, durch Kurzschließen des ersten teilweise deaktivierten Katalysatorbetts des Hauptreaktors, in das nächste nicht-deaktivierte Katalysatorbett des Hauptreaktors, das unmittelbar stromabwärts in Bezug auf die Zirkulationsrichtung der Charge angeordnet ist,
- ein Schritt c), in dem die Charge nur und aufeinanderfolgend alle Katalysatorbetten des Hauptreaktors durchquert,
- ein Schritt c'), gleichzeitig mit dem Schritt c), in dem der deaktivierte Katalysator des oder der Katalysatorbetten des Schutzbett-Reaktors regeneriert wird und/oder durch einen frischen Katalysator ersetzt wird,
- ein Schritt d), in dem die Charge nur den Schutzbett-Reaktor durchquert,
- ein Schritt d'), gleichzeitig mit dem Schritt d), in dem der deaktivierte Katalysator der mindestens zwei Katalysatorbetten des Hauptreaktors regeneriert wird und/oder durch einen frischen Katalysator ersetzt wird;
wobei während des Verfahrens die Betriebsdauer für jeden der Schritte a), b), c) und d) in Bezug auf eine maximale Betriebsdauer tₘₐₓ für jeden Schritt definiert wird, welche die Zeit ist, um einen maximal tolerierbaren Wert einer Temperatur oder einer Selektivität für eine gegebene Charge zu erreichen, und jeweils beträgt:
• für den Schritt a): zwischen 10 und 70 % der t_{Amax},
• für den Schritt b): zwischen 40 und 100 % der t_{Bmax},
• für den Schritt c): zwischen 90 und 100 % der t_{Cmax},
• für den Schritt d): zwischen 70 und 100 % der t_{Dmax},
mit der Maßgabe, dass:
• der maximal tolerierbare Wert einer Charge C3 die Selektivität ist, die dem Erreichen einer Gleichheit der Propylenzusammensetzung zwischen dem Eingang und dem Ausgang des Reaktors am Zyklusende entspricht;
• der maximal tolerierbare Wert einer Charge C4 eine maximale Temperatur von 160 °C ist;
• der maximal tolerierbare Wert einer Charge C5 eine maximale Temperatur von 160 °C ist;
• der maximal tolerierbare Wert einer Charge von Pyrolysebenzin eine maximale Temperatur von 200 °C ist.

2. Verfahren nach Anspruch 1, wobei das Gesamtvolumen des Katalysators, der in dem oder den Katalysatorbetten des Schutzbett-Reaktors enthalten ist, maximal 60 % des Gesamtvolumens des Katalysators beträgt, der in den Katalysatorbetten des Hauptreaktors enthalten ist.

3. Verfahren nach Anspruch 1, wobei ein Schritt a') durchgeführt wird, gleichzeitig mit dem Schritt a), in dem der während des Schritts d) deaktivierte Katalysator des oder der Katalysatorbetten des Schutzbett-Reaktors regeneriert wird und/oder durch einen frischen Katalysator ersetzt wird.

4. Verfahren nach einem der vorhergehenden Ansprüche, wobei jeder Reaktor bei einer Temperatur von 0 °C bis 200 °C, bei einem Druck zwischen 1 und 6,5 MPa und bei einer globalen Volumengeschwindigkeit pro Stunde, definiert als das Verhältnis des Volumendurchsatzes der frischen Charge bei 15 °C zum Gesamtvolumen des Katalysators, der in der Gesamtheit der verwendeten Reaktoren vorhanden ist, zwischen 1 h⁻¹ und 100 h⁻¹ betrieben wird.

5. Verfahren nach einem der vorhergehenden Ansprüche, wobei ein Teil des Abflusses aus dem Hauptreaktor oder dem Schutzbett-Reaktor in Mischung mit der zu hydrierenden Charge rezykliert wird.

6. Verfahren nach einem der vorhergehenden Ansprüche, wobei die Betriebsbedingungen der Hydrierung während der Schritte a), b), c) und d) identisch sind.

7. Verfahren nach einem der Ansprüche 1 bis 5, wobei während des Schritts d) die Temperatur des Schutzbett-Reaktors erhöht wird und/oder der Strom der Phase, umfassend Wasserstoff, erhöht wird, und/oder der Durchsatz der Rezyklierung in dem Schutzbett-Reaktor erhöht wird und/oder der Durchsatz der Charge, die in den Schutzbett-Reaktor eingebracht wird, verringert wird, jeweils im Verhältnis zu der Temperatur, dem Strom und/oder dem/den Durchsatz/Durchsätzen, der/die am Beginn von Schritt a) verwendet wird/werden.

8. Verfahren nach Anspruch 7, wobei die Erhöhung der Temperatur des Schutzbett-Reaktors am Kopf des Reaktors am Beginn von Schritt d) im Verhältnis zum Beginn von Schritt a) zwischen 0,5 und 40 °C beträgt.

9. Verfahren nach den Ansprüchen 7 oder 8, wobei die Erhöhung des Rezyklierungsdurchsatzes zum Schutzbett-Reaktor am Beginn von Schritt d) im Verhältnis zum Rezyklierungsdurchsatz am Beginn von Schritt a) zwischen 0,5 und 100 % beträgt.

10. Verfahren nach einem der vorhergehenden Ansprüche, wobei ein Abschreckmittel zwischen zwei Katalysatorbetten in dem Hauptreaktor oder dem Schutzbett-Reaktor eingebracht wird, wobei das Abschreckmittel ein flüssiges Abschreckmittel und/oder ein gasförmiges Abschreckmittel sein kann.

11. Verfahren nach einem der vorhergehenden Ansprüche, wobei ein oder mehrere Wärmetauscher zwischen dem Schutzbett-Reaktor und dem Hauptreaktor verwendet wird oder werden.

12. Verfahren nach einem der vorhergehenden Ansprüche, wobei die verwendeten Hydrierungskatalysatoren in dem oder den Katalysatorbetten des Hauptreaktors und/oder des Schutzbett-Reaktors identisch oder verschieden sind.

## Claims

1. A process for the selective hydrogenation of a feed of hydrocarbons containing polyunsaturated molecules comprising at least 3 carbon atoms and selected from the group constituted by a C3 cut from steam cracking, a C4 cut from steam cracking, a C5 cut from steam cracking and a pyrolysis gasoline, and a mixture thereof, in which said feed and a gaseous phase comprising hydrogen are passed, under hydrogenation conditions, over a hydrogenation catalyst in a single principal fixed bed reactor containing at least two catalytic beds and a fixed bed guard reactor which is smaller in size containing at least one catalytic bed, said reactors being disposed in series for use in a cyclic manner by repeating the steps a), b), c), c'), d) and d') defined below in succession:
- a step a), during which the feed passes in succession through all of the catalytic beds of the principal reactor,
- when the first catalytic bed of the principal reactor starts to become deactivated, a step b), during which the feed is introduced into the guard reactor then, by short-circuiting the first partially deactivated catalytic bed of the principal reactor, into the next non-deactivated catalytic bed of said principal reactor located immediately downstream with respect to the direction of movement of the feed,
- a step c), during which the feed passes uniquely and successively through all of the catalytic beds of the principal reactor,
- a step c'), simultaneously with step c), during which the deactivated catalyst of the catalytic bed or beds of the guard reactor is regenerated and/or replaced with fresh catalyst,
- a step d), during which the feed only passes through the guard reactor,
- a step d'), simultaneously with step d), during which the deactivated catalyst of the at least two catalytic beds of the principal reactor is regenerated and/or replaced with fresh catalyst;
process in which the period of operation for each of steps a), b), c) and d), defined with respect to a maximum operating period tmax for each step, which is the time to reach a maximum tolerable value for a temperature or a selectivity for a given feed, is respectively:
• for step a): between 10% and 70% of t_{Amax}
• for step b): between 40% and 100% of t_{Bmax}
• for step c): between 90% and 100% of t_{Cmax}
• for step d): between 70%and 100% of t_{Dmax} ;
and in which
• the maximum tolerable value for a C3 feed is the selectivity in accordance with the specifications in force;
• the maximum tolerable value for a C4 feed is a maximum temperature of 160°C;
• the maximum tolerable value for a C5 feed is a maximum temperature of 160 °C;
• the maximum tolerable value for a feed of pyrolysis gasoline is a maximum temperature of 200°C.

2. The process as claimed in claim 1, in which the total volume of catalyst contained in the catalytic bed or beds of the guard reactor is a maximum of 60% of the total volume of catalyst contained in the catalytic beds of the principal reactor.

3. The process according to claim 1, in which a step a') is carried out simultaneously with step a), during which the catalyst deactivated during step d) of the catalytic bed or beds of the guard reactor is regenerated or replaced with fresh catalyst.

4. The process as claimed in one of the preceding claims, in which each reactor is operated at a temperature of 0°C to 200°C, at a pressure in the range 1 to 6.5 MPa and at an overall hourly space velocity, defined as the ratio of the volume flow rate of the fresh feed at 15°C to the total volume of catalyst present in the series of reactors employed, in the range 1 h⁻¹ to 100 h⁻¹.

5. The process as claimed in one of the preceding claims, in which a portion of the effluent obtained from the principal reactor or from the guard reactor is recycled as a mixture with the feed to be hydrogenated.

6. The process as claimed in one of the preceding claims, in which the operating conditions for hydrogenation during steps a) b) c) and d) are identical.

7. The process as claimed in one of claims 1 to 5, in which during step d), the temperature of the guard reactor is increased and/or the flow of the phase comprising hydrogen is increased, and/or the recycle flow rate into the guard reactor is increased and/or the flow rate of feed introduced into the guard reactor is reduced with respect to respectively the temperature, the flow or the flow rate(s) employed at the start of step a).

8. The process as claimed in claim 7, in which the increase in the temperature of the guard reactor at the head of the reactor at the start of step d) with respect to the start of step a) is in the range 0.5°C to 40°C.

9. The process as claimed in claim 7 or claim 8, in which the increase in the recycle flow rate at the guard reactor at the start of step d) with respect to the recycle flow rate at the start of step a) is in the range 0.5% to 100%.

10. The process as claimed in one of the preceding claims, in which a quench is introduced between two catalytic beds in the principal reactor or the guard reactor, said quench possibly being a liquid quench and/or a gaseous quench.

11. The process as claimed in one of the preceding claims, in which one or more heat exchanger(s) is employed between the guard reactor and the principal reactor.

12. The process as claimed in one of the preceding claims, in which the hydrogenation catalysts used are identical or different in the catalytic bed or beds of the principal reactor and/or of the guard reactor.
